# EUROPEAN PATENT SPECIFICATION

(11) **EP 4 536 187 B1**
(45) Date of publication and mention of the grant of the patent: **28.01.2026**
(21) Application number: 23730517.2
(22) Date of filing: 05.06.2023
(51) Int. Cl.: A61K 9/00, A61K 9/08, A61K 9/19, A61K 31/519, A61K 47/02, A61P 35/00

(54) **COMPOSITIONS COMPRISING DISODIUM 5,10-METHYLENE-(6R)-TETRAHYDROFOLATE**
ZUSAMMENSETZUNGEN MIT DINATRIUM-5,10-METHYLEN-(6R)-TETRAHYDROFOLAT
COMPOSITIONS COMPRENANT DU 5,10-MÉTHYLÈNE-(6R)-TÉTRAHYDROFOLATE DISODIQUE

(30) Priority: 08.06.2022 EP 22177932
(43) Date of publication of application: 16.04.2025
(73) Proprietor: Merck Patent GmbH, 64293 Darmstadt (DE)
(72) Inventor: MOSER, Rudolf, 6460 ALTDORF / URI (CH); GROEHN, Viola, 6460 ALTDORF / URI (CH); AMMANN, Thomas, 6460 ALTDORF / URI (CH); KNAPP, Jean-Pierre, 6460 ALTDORF / URI (CH); SVAERD, Marianne, 41346 GOTHENBURG (SE)
(74) Representative: Merck Patent Association
(86) International application number: PCT/EP2023/064972
(87) International publication number: WO 2023/237484

(56) References cited:
- CN-A- 107 892 692
- JP-A- 2020 531 415
- RU-C2- 2 343 923
- US-A- 5 300 505
- US-A- 5 997 915
- US-A1- 2009 221 594
- US-A1- 2022 024 940
- US-B2- 10 570 134

## Description

The present invention relates to stable formulations and lyophilizates comprising a high content of the disodium salt of 5,10-methylene-(6R)-tetrahydrofolic acid (5,10-CH₂-(6R)-THF*Na₂), sulfate and citrate.

### BACKGROUND OF THE INVENTION

5,10-methylenetetrahydrofolic acid is known as a medicament used in combination with 5-fluorouracil (5-FU) in the treatment of solid tumors (Seley, K. L. Drugs 4 (1), 99, 2001). The active isomeric form 5,10-methylene-(6R)-tetrahydrofolic acid (referred to as 5,10-CH₂-(6R)-THF in the following), achieves its chemotherapeutic effect together with the base analogue and 5-FU metabolite 5-FdUMP by inhibiting the enzyme thymidylate synthase (TS). TS catalyzes the conversion of deoxyuridylate (dUMP) to deoxythymidylate (dTMP), which is an essential building block for DNA synthesis. Deactivation of TS occurs by formation of a covalent, ternary inhibition complex between TS, the base analogue 5-FdUMP, which is a metabolite of 5-FU, and 5,10-CH₂-(6R)-THF.

An enhancement of the cytotoxic effect of 5-FU can be achieved by increasing the intracellular concentration of 5,10-CH₂-(6R)-THF, whereupon the stability of the ternary inhibition complex is increased. This causes direct inhibition of DNA synthesis and repair, which ultimately results in cell death and delay of tumor growth. In order to achieve high intracellular concentrations of 5,10-CH₂-(6R)-THF the application of respective stable, high content products is desired.

However, there are undesirable properties associated with 5,10-CH₂-(6R)-THF that limit its pharmaceutical use. For example, 5,10-CH₂-(6R)-THF is highly susceptible to oxidation and chemical degradation that results in unfavorably high impurity levels.

Susceptibility to oxidation and chemical degradation of 5,10-CH₂-(6R)-THF is especially high in aqueous solution, or when the compound is present in its amorphous form where it has a large surface (e.g. in its pharmaceutical use form as a lyophilizate), or in re-dissolved form such as solutions for injection. It is well known that to be amenable for pharmaceutical use, the respective composition needs to fulfil several requirements including high chemical and isomeric stability, such that effective storage over an acceptable period of time can be achieved, without exhibiting a significant change in the composition's physicochemical characteristics, ease of handling and processing, etc..

5,10-methylenetetrahydrofolic acid is an addition product of tetrahydrofolic acid and formaldehyde (see e.g. Poe, M. et al. Biochemistry 18 (24), 5527, 1979; Kallen, R. G. Methods in Enzymology 18B, 705, 1971) and is known for its extremely high sensitivity to oxidation by air as well as instability in neutral and/or acidic environments, potentially leading to chemical degradation and/or hydrolysis (see e.g. Odin, E. et al., Cancer Investigation 16 (7), 447, 1998; Osborn, M. J. et al., J. Am. Chem. Soc. 82, 4921, 1960; Hawkes, J., and Villota, R. Food Sci. Nutr. 28, 439, 1989).

Attempts to stabilize compositions of 5,10-methylenetetrahydrofolates have included e.g. (i) rigorous exclusion of atmospheric oxygen by the use of special technical devices for the reconstitution of solid formulations and the injection of 5,10-methylenetetrahydrofolates in an air-free environment (see e.g. Odin, E. et al., Cancer Investigation 16 (7), 447, 1998; U.S. Pat. No. 4,564,054); (ii) addition of a reducing agent such as L(+)-ascorbic acid or salts thereof, reduced gamma-glutathione, beta-mercaptoethanol, thioglycerol, N-acetyl-L-cysteine, etc. as an antioxidant for the highly sensitive 5,10-methylenetetrahydrofolic acid and for tetrahydrofolic acid in particular.

As an example of the effectiveness of reducing agents (antioxidants) in the prevention of oxidation of the 5,10-methylenetetrahydrofolic acid molecule, the company Adventrx Pharmaceuticals carried out stability studies on their drug candidate CoFactor^{®}, i.e. the calcium slat of the diastereomer mixture5,10-methylene-(6R,S)-tetrahydrofolic acid, which studies were disclosed *i.a*. in WO 2007/064968. The chemical stability of the diastereomer mixture 5,10-methylene-(6R,S)-tetrahydrofolic acid is assumed to be similar to the pure diastereomer 5,10-CH₂-(6R)-THF of the present invention.

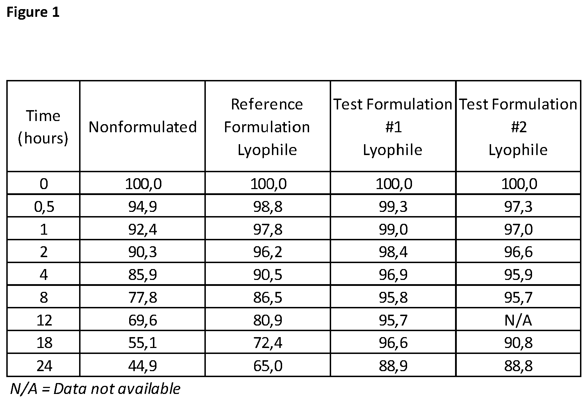

The Adventrx study compared the stability of 5,10-methylenetetrahydrofolic acid as such (non-formulated) or formulated with trisodium citrate alone or formulated with both trisodium citrate and ascorbic acid (see Figure 1).

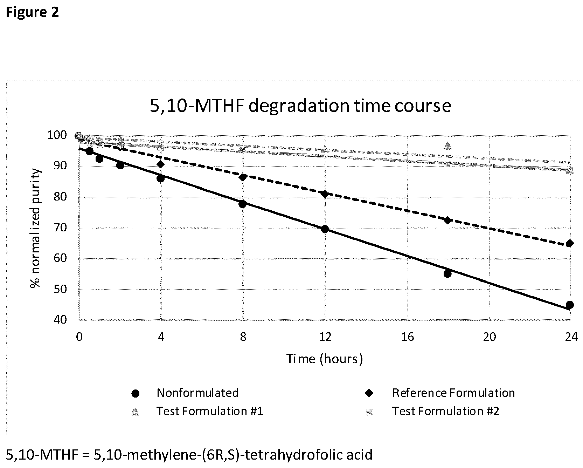

Linear regression analysis of the stability profiles of the isolated lyophilizates showed that degradation of 5,10-methylene-(6R,S)-tetrahydrofolic acid was linear over time (see Figure 2). The degradation rate (slope of the best-fit line) for each formulation (re-constituted lyophilizate) demonstrated the following order, from fastest to slowest degradation rate: nonformulated > formulated with only trisodium citrate > formulated with both trisodium citrate and the reducing agent ascorbic acid (Figure 2). Nonformulated 5,10-methylene-(6R,S)-tetrahydrofolic acid was thus found to loose 2.2% purity per hour, resulting in a purity of 84% after 7 hours, whereas formulations containing 250 % w/w trisodium citrate was found to loose 1.4 % purity per hour, resulting in a purity of 89% after 7 hours. Formulations containing both 250 % w/w trisodium citrate and 175 % w/w ascorbic acid had much higher stability, loosing only 0.5% purity per hour, resulting in a purity of about 96% after 7 hours.

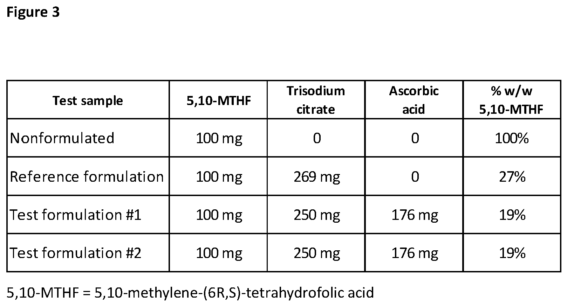

The study thus showed that the addition of a reducing agent has a major stabilising effect on formulations of 5,10-methylenetetrahydrofolic acid. This, however, has the consequence that the content of the active ingredient in the lyophilized formulation is reduced to less than 20% w/w (Figure 3). Solutions disclosed in WO 2007/064968 for the purpose of preparing said lyophilizates contain at most about 4% by weight 5,10-CH₂-THF.

Stabilization of 5,10-methylenetetrahydrofolic acid has also been achieved by formation of various crystalline forms such as the sulfate salts (see e.g. EP 0 537 492) or hemisulfate salts (see e.g. EP 2 837 631). However, such salt forms of 5,10-methylenetetrahydrofolic acid are not readily useful for pharmaceutical purposes due to their low aqueous solubility.

Lyophilizates of 5,10-CH₂-(6R)-THF containing dicarboxylic acids and/or tricarboxylic acids such as citric acid and/or other stabilizers have also been disclosed in e.g. WO 2019/034673, US 2007/0099866 and US10059710 B2. Solutions disclosed therein for the purpose of preparing lyophilizates contain at most 2-3% by weight 5,10-CH₂-(6R)-THF.

From a clinical perspective the formulations of 5,10-CH₂-(6R)-THF known in the art do not have a satisfactory combination of aqueous solubility, high content of the active ingredient and high stability. There thus still remains a great need for stable and soluble pharmaceutical compositions with a high content of 5,10-CH₂-(6R)-THF.

### SUMMARY OF THE INVENTION

It has now surprisingly been found that lyophilized compositions which comprise the disodium salt of 5,10-CH₂-(6R)-methylene tetrahydrofolic acid (denoted hereinafter 5,10-CH₂-(6R)-THF*Na₂) in combination with citrate and an alkali metal sulfate have comparable or even higher stability than compositions comprising 5,10-methylenetetrahydrofolic acid, citrate and reducing agents such as L-(+)-ascorbic acid.

Thus, even without additions of reducing agents (antioxidants), such as L-(+)-ascorbic acid, and without the exclusion of atmospheric oxygen, solutions of 5,10-CH₂-(6R)-THF*Na₂, alkali metal sulfate and citrate remain highly stable for hours, which solutions according to the present invention are next converted into lyophilizates with a similar good stability. These lyophilizates comprise 5,10-CH₂-(6R)-THF*Na₂, alkali metal sulfate and citrate and no other stabilizing agents, and thus overcome the previously discussed known drawbacks, and allow for the preparation of solid-state pharmaceutical compositions of high purity and a low content of either oxidation products or other chemical degradation products.

In a first aspect the present invention thus relates to a lyophilized composition which comprises the disodium salt of 5,10-methylene-(6R)-tetrahydrofolic acid (5,10-CH₂-(6R)-THF*Na₂), citrate and an alkali metal sulfate, which composition does not contain any further reducing agents or antioxidants, and wheren the concentration of said disodium salt of 5,10-methylene-(6R)-tetrahydrofolic acid is more than 30% w/w.

A second aspect of the present invention is directed to a process for the preparation of the lyophilized composition according to the first aspect, which process comprises the following steps:
i. dissolving (6S)-tetrahydrofolic acid in water at pH 11,
ii. adjusting the pH of the clear solution to 8.6 ± 0.5
iii. adding 110 - 120 mol% formaldehyde,
iv. stirring the reaction mixture until reaction has completed,
v. adding from 40 - 200 mol-% of an alkali metal sulfate and 100 - 400 mol-% of citrate,
vi. filtering the reaction mixture to obtain a clear solution, and
vii. freeze-drying the obtained clear solution,
which process does not comprise adding any further reducing agents or antioxidants, and wherein the concentration of said disodium salt of 5,10-methylene-(6R)-tetrahydrofolic acid in the lyophilized composition is more than 30% w/w.

In a third aspect there is disclosed a lyophilized composition according to the first aspect for use in the treatment of cancer, or in cancer therapy, in a human patient.

In a fourth aspect there is disclosed a method of treatment of cancer, or of cancer therapy, in human patients comprising administering a lyophilized composition according to the first aspect to a human patient in need thereof.

In a fifth aspect there is disclosed the use of a lyophilized composition according to the first aspect for the manufacture of a medicament for the treatment of cancer in human patients.

### BRIEF DESCRIPTION OF THE FIGURES

**Figure 1** **is** a table adapted from WO 2007/064968 which demonstrates the stability over time of non-formulated and various formulated forms of 55,10-methylene-(6R,S)-tetrahydrofolic acid (% normalized purity). Nonformulated 5,10-methylene-(6R,S)-tetrahydrofolic acid was found to loose 2.2% purity per hour, resulting in a normalized purity of 45% after 24 hours. A reference formulation containing 250 % w/w trisodium citrate was found to loose 1.4% purity per hour, resulting in a normalized purity of 65% after 24 hours. Formulations #1 and #2 containing both 250 % w/w trisodium citrate and 175 % w/w ascorbic acid had a much higher stability, loosing only 0.5% purity per hour, resulting in a normalized purity of about 90% after 24 hours.
**Figure 2** is adapted from Figure 1 in WO 2007/064968 and demonstrates graphically the tabulated results of Fig. 1 herein.
**Figure 3** is a table adapted from Example 1 of WO 2007/064968 showing the composition of the non-formulated and formulated forms of 5,10-methylene-(6R,S)-tetrahydrofolic acid shown in Fig. 1 and Fig. 2 herein. As can be seen, the most stable compositions (formulations #1 and #2) contain less than 20% of the active ingredient 5,10-methylene-(6R,S)-tetrahydrofolic acid.

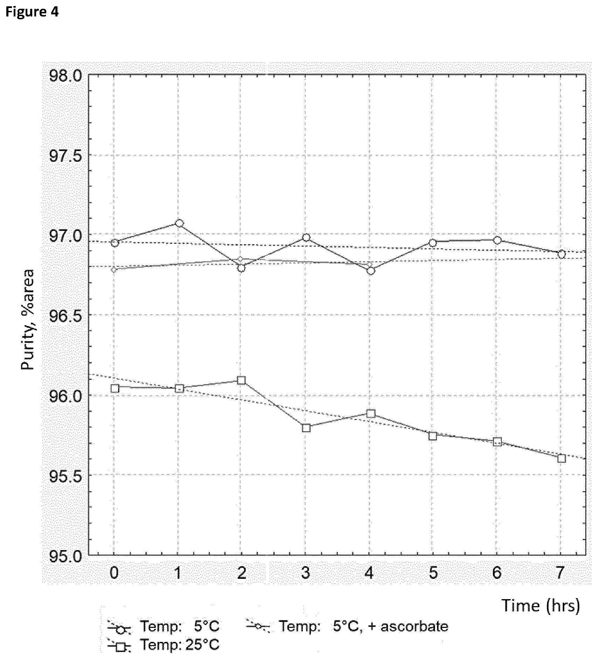

**Figure 4** shows the purity analyses of three solutions comprising 75 mg/mL of the disodium salt of 5,10-methylene-(6R)-tetrahydrofolic acid, citrate and sodium sulfate tested at three different conditions: 25 °C, 5 °C and 5 °C with 1 mg/mL sodium ascorbate added as antioxidant.
   The results are shown for a total period of 7 hours. As can be seen from the graphs, the solutions are very stable under the storage conditions. At 5 °C there is practically no change in purity, which remains at about 97% (area%). As can also be seen, the effect of ascorbate is minimal. At 25 °C, the purity over 7 hours changes from an initial purity of about 96% to a purity of about 95.5% (area%).

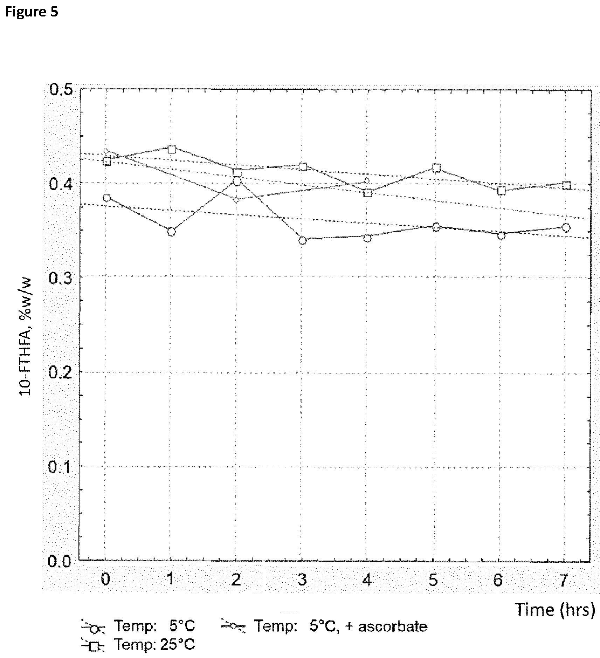

**Figure 5** shows analyses of the same three solutions of sodium salt of 5,10-methylene-(6R)-tetrahydrofolic acid as shown in Figure 4 herein. In Figure 5, the development over 7 hours of the main impurity, 10-formyl-(6R)-tetrahydrofolic acid (10-FTHFA) in the solutions is shown. As can be seen, the level of this impurity is practically constant over time.

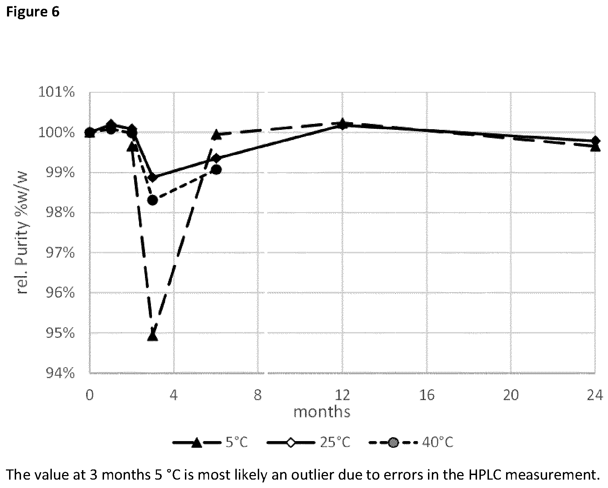

**Figure 6** shows the stability of a lyophilized composition comprising disodium 5,10-methylene-(6R)-tetrahydrofolate, citrate and sodium sulfate at at 5 °C, 25°C, and 40°C. As can be seen from the graphs, the lyophilized compositions are very stable under the storage conditions. Even at room temperature there is practically no change in purity when stored for 24 months (99.8% relative purity %w/w).

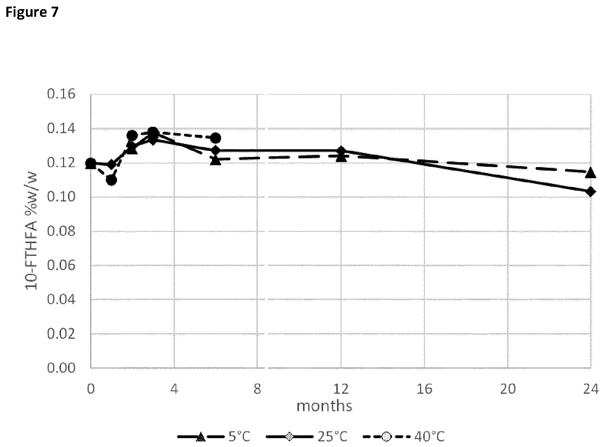

**Figure 7** shows the the development of the main impurity, 10-formyl-(6R)-tetrahydrofolic acid (10-FTHFA), in a lyophilized composition comprising disodium 5,10-methylene-(6R)-tetrahydrofolate, citrate and sodium sulfate when stored at at 5 °C, 25°C, and 40 °C. As can be seen, the level of this impurity is practically constant over time.

### DEFINITIONS

In the present text, the term "stabilizers" or "stabilizing agents" relates to buffers such as citrate (or citric acid and salts thereof); dicarboxylates such as succinate, malate and maleate; tris(hydroxymethyl)aminomethane (TRIS); N-tris(hydroxymethyl)methyl-2-aminoethanesulfonic acid (TES); 3-(N-morpholino)propanesulfonic acid (MOPS); N,N-bis(2-hydroxyethyl)-2-aminoethanesulfonic acid (BES); MES; MOPSO; HEPES; phosphate; carbonate; ammonium; mono-, di-, and tri-alkylammonium; mono-, di-, and tri-hydroxylalkylammonium; glutamate; borate; lactate, as well as combinations of these. The term "stabilizers" or "stabilizing agents" further relates to reducing agents such as L-(+)ascorbic acid or salts thereof, reduced γ-glutathione, β-mercaptoethanol, thioglycerol, N-acetyl-L-cysteine, etc. which may act as an antioxidant for the sensitive 5,10-methylenetetrahydrofolic acid, and for the tetrahydrofolic acid in particular.

In the present text, the term "buffer" relates to citrate (or citric acid and salts thereof), dicarboxylates such as succinate, malate and maleate, tris(hydroxymethyl)aminomethane (TRIS); N-tris(hydroxymethyl)methyl-2-aminoethanesulfonic acid (TES); 3-(N-morpholino)propanesulfonic acid (MOPS); N,N-bis(2-hydroxyethyl)-2-aminoethane-sulfonic acid (BES); MES; MOPSO; HEPES; phosphate; carbonate; ammonium ; mono-, di-, and tri-alkylammonium; mono-, di-, and tri-hydroxylalkylammonium; glutamate; borate; lactate, as well as combinations of these.

In the present text, the term "reducing agent" or "antioxidants" relates to L-(+)ascorbic acid or salts thereof, reduced γ-glutathione, β-mercaptoethanol, thioglycerol, and N-acetyl-L-cysteine.

In the present text, the term "solvent" relates to solvents which may be used in freeze drying processes. "Solutions" as referred to in the present text, comprise aqueous solutions as well as solutions in organic solvents. Typically, "aqueous solutions" mean solutions in water, saline solutions, water containing small amounts of buffers, water containing isotonic amounts of NaCl, or mixtures of water with organic solvents, and the like. Typical organic solvents include DMSO, acetonitrile, acetone, methanol, or ethanol.

In the present text, the phrase "composition comprising 5,10-CH₂-(6R)-THF*Na₂" relates to both solutions and solid compositions, such as lyophilizates, including lyophilizates which have been reconstituted, eg. for use in medical treatment.

### DETAILED DESCRIPTION OF THE INVENTION

It is well-known in the art that 5,10-methylenetetrahydrofolic acid is extremely sensitive to oxidation. It is furthermore known that solutions of 5,10-methylenetetrahydrofolic acid are more susceptible to chemical degradation than solid forms thereof. Previously, this problem has been addressed in the art by adding antioxidants, i.e., reducing agents such as ascorbate, to solutions 5,10-methylenetetrahydrofolic acid already containing citric acid. This was shown to increase the stability of dissolved 5,10-methylenetetrahydrofolic acid as shown in Figure 1 and Figure 2 herein, but at the cost of decreasing the relative content of 5,10-methylenetetrahydrofolic acid.

It has now surprisingly been found that aqueous solutions comprising the disodium salt of 5,10-methylene-(6R)-tetrahydrofolic acid (denoted hereinafter 5,10-CH₂-(6R)-THF*Na₂) in combination with citrate and an alkali metal sulfate have comparable or even higher stability than aqueous solutions comprising 5,10-methylenetetrahydrofolic acid, citrate and reducing agents such as L-(+)-ascorbic acid, which solutions according to the present invention are next converted into lyophilizates with a similar good stability. Alkali metal sulfates are not reducing agents *per se* and are therefore normally considered chemically inert. The found stability is therefore surprising.

Lyophilizates of the present invention contain more than 30% w/w, such as more than 35% w/w, or such as about 40 % w/w 5,10-CH₂-(6R)-THF*Na₂.

The lyophilized powders have even higher stability than the aqueous solutions and can be reconstituted with a diluent to a set concentration for administration. Such reconstituted lyophilizates can be administered either intramuscularly or intravenously.

Bulking agents such as mannitol may be added to the solutions before the freeze-drying process to promote an acceptable lyophilized cake formation. The structure and porosity of the lyophilized cake is important, since good pore formation may facilitate drying and transport of the water during the drying cycle.

Also, electrolytes, sugars and/or polyols such as dextrose, glycerol, mannitol and sodium chloride may be added to adjust the osmolality. Osmolality adjustment can be done before or after freeze drying. The reconstituted lyophilisate solution preferably has an osmolality in the range of 250- 350 mOsm. However, an osmolality of 200 - 600 mOsm can be tolerated as well, and will depend on the volume to be administered as well as the injection/infusion time.

The pH of the solutions is typically in the range of 8.0 to 9.0, preferably in the range of 8.4 to 8.8 and can be adjusted during drug product manufacturing with e.g. small amounts of hydrochloric acid or sodium hydroxide.

Stability is a critical property and component of pharmaceutical formulation studies and drug development. Stability studies are performed both in solution and solid state. It is an established fact that the solution state and solid-state stability can differ both qualitatively and quantitatively. Extensive studies were performed for stability of the drug substance and pharmaceutical compositions thereof by exposing it to variety of stressors, like high temperature and/or high humidity. These studies also provide information on the degradation products and help in developing meaningful specifications as well as the intrinsic stability of the pharmaceutical composition. Most common pathways for drug degradation include i.a. hydrolysis, oxidation, and photochemical degradation.

The purpose of stability testing is to provide evidence on how the quality of a product varies with time under the influence of a variety of environmental factors such as temperature, humidity, and light, and to establish a suitable shelf life for the pharmaceutical product and recommended storage conditions, in order to ensure patient safety.

In a first aspect the present invention relates to a lyophilized composition comprising the disodium salt of 5,10-methylene-(6R)-tetrahydrofolic acid (5,10-CH₂-(6R)-THF*Na₂), citrate and an alkali metal sulfate, which composition does not contain any further reducing agents or antioxidants, and wherein the concentration of said disodium salt of 5,10-methylene-(6R)-tetrahydrofolic acid is more than 30% w/w.

In a preferred embodiment of the first aspect the present invention relates to stable lyophilizates which contain more than 35% w/w, or such as about 40% w/w 5,10-CH₂-(6R)-THF*Na₂.

Lyophilizates of the present invention are substantially amorphous while having an enhanced stability, such as an enhanced storage stability. Lyophilizates of the present invention may by methods known in the art be reconstituted into aqueous pharmaceutical formulations to be administered into a patient in need thereof.

The present invention in one embodiment discloses a lyophilized composition according to the first aspect wherein the molar ratio of alkali metal sulfate to 5,10-CH₂-(6R)-THF*Na₂ is from 40 mol-% to 400 mol-%, preferably from about 50 mol-% to about 100 mol-%.

In another embodiment the present invention discloses a lyophilized composition according to the first aspect wherein the molar ratio between 5,10-CH₂-(6R)-THF*Na₂ and citrate is from 200 - 400 mol%, preferably around 350 mol%.

A second aspect of the present invention is directed to a process for the preparation of the lyophilized composition according to the first aspect, which process comprises the following steps:
i. dissolving (6S)-tetrahydrofolic acid in water at pH 11,
ii. adjusting the pH of the clear solution to 8.6 ± 0.5,
iii. adding 110 - 120 mol% formaldehyde,
iv. stirring the reaction mixture until reaction has completed,
v. adding from 40 - 200 mol-% of an alkali metal sulfate and 100 - 400 mol-% of citrate,
vi. filtering the reaction mixture to obtain a clear solution, and
vii. freeze-drying the obtained clear solution,
which process does not comprise adding any further reducing agents or antioxidants, and wherein the concentration of said disodium salt of 5,10-methylene-(6R)-tetrahydrofolic acid in the lyophilized composition is more than 30% w/w.

In an embodiment, the alkali metal sulfate added in step v. is in the form of sodium sulfate. In an embodiment, the citrate added in step v. is in the form of sodium citrate. In another embodiment, the citrate is added as citric acid.

In a third aspect there is disclosed a lyophilized composition comprising 5,10-CH₂-(6R)-THF*Na₂ according to the first aspect for use in the treatment of cancer, or in cancer therapy, in a human patient.

In a preferred embodiment the present invention relates to a stable lyophilizate comprising 5,10-CH₂-(6R)-THF*Na₂ according to the first aspect, or a reconstituted aqueous solution thereof, for use in the treatment of cancer, or in cancer therapy, in a human patient.

In a fourth aspect there is disclosed a method of treatment of cancer, or of cancer therapy, in human patients comprising administering a lyophilized composition according to the first aspect, to a human patient in need thereof.

In a preferred embodiment there is disclosed a method of treatment of cancer in human patients comprising administering a lyophilized composition comprising 5,10-CH₂-(6R)-THF*Na₂ according to the first aspect, or a reconstituted aqueous solution thereof, to a human patient in need thereof.

In a fifth aspect there is disclosed the use of a lyophilized composition comprising 5,10-CH₂-(6R)-THF*Na₂ according to the first aspect for the manufacture of a medicament for the treatment of cancer in human patients.

A further aspect is directed to reconstituted pharmaceutical compositions of the lyophilisates of the present invention comprising 5,10-CH₂-(6R)-THF*Na₂ and a pharmaceutically acceptable carrier or diluent, such as sterile water or a liquid pharmaceutically acceptable vehicle, optionally further comprising at least one additional therapeutic agent including but not limited to, bactericides, antibiotics, antivirals, antiseptics, antineoplastics, anticancer compounds such as chemotherapeutic agents, antifungals, and/or anti-inflammatory agents or other bioactive or therapeutic agents that are suitable for human use, in particular anticancer compounds such as chemotherapeutic agents, for example 5-FU and derivatives, and antifolates, e.g. methotrexate, Pemetrexed.

The lyophilizates of the invention are in substantially unsolvated, anhydrous form, which includes compounds that are completely free of water and compounds which may contain traces of water. Such possible residual (not stoichiometric) amounts of water may be any amount of water, but typically ranges from 0 wt.-% H₂O to 3 wt.-% H₂O, preferably between 0 wt.-% H₂O to 1 wt.-% H₂O, such as between 0.05 wt.-% H₂O to 1 wt.-% H₂O.

### EXAMPLES

### HPLC

For the measurement of purity/content and degradation products an HPLC-UV Gradient Method was used: Column type: ODS, Mobile phase: A: aqueous Buffer; Mobile Phase: B: aqueous Buffer/Methanol, Run time: 30min, Sample Solvent: aqueous Buffer.

### Water content

The determination of the water content was made according to Ph. Eur. 2.5.32/USP <921/ Method Ic>.

### Osmolality

The determination of the osmolality was made according to Ph. Eur. 2.2.35 (osmometer)/USP <785>.

### Example 1: Preparation of an aqueous solution comprising disodium 5,10-methylene-(6R)-tetrahydrofolate, citrate and sodium sulfate

(a) (6S)-tetrahydrofolic acid (16 mmol, 7.93 g) was dissolved in 78.0 g distilled water at room temperature under nitrogen. The pH of the solution was first raised to pH 11 by slow addition of a 32% NaOH solution and then adjusted with 1.0 M HCl to pH 8.3 and cooled to about 0°C. A formaldehyde solution (1.44 g, 110 mol %) was added in one portion, and the solution was stirred at 0°C for 1 hour. Active charcoal (0.2 g, Norit C Extra) was added, and the reaction mixture was stirred for 30 minutes at 0°C and filtered to obtain a clear solution of disodium 5,10-methylene-(6R)-tetrahydrofolate (5,10-CH₂-(6R)-THF*Na₂).
   The so obtained solution of disodium 5,10-methylene-(6R)-tetrahydrofolate may be optionally be purified by the following steps (b) and (c) by precipitation of a sulfate salt of 5,10-CH₂-(6R)-THF followed by redissolution with sodium hydroxide.
(b) (Optional) 55 ml 1M H₂SO₄ (0.055 mol; 344 mol%) was heated to 60°C under nitrogen, and the solution as obtained in above step (a) was added dropwise over 15 minutes. The obtained reaction mixture (slurry) was stirred at 50°C for 2 hours. The reaction mixture was then suction filtered at 50°C, and the precipitate washed twice with 25 ml distilled water at room temperature and dried at 30°C and 10 mbar for 12 hours (overnight) to obtain 7.36 g 5,10-CH₂-(6R)-THF sulfate as light gray crystals (86% yield).
(c) (Optional) The product of step (b) was dissolved in 50 mL 0.1 N NaOH under nitrogen, and the pH was adjusted to 8.6 ±0.50 with 1.5 N NaOH.
(d) 21 gr citric acid in 20 ml water was added to the solution in step (c) or step (a), and adjustment of the sulfate content up to 200 mol% was performed with aqueous sodium sulfate if necessary. The total volume was then adjusted to 100 mL with sterile filtered water and filtered using a 0.22 µm filter, preferably a hydrophilic PVDF type e.g. Millipore Durapore^{®} 0.22µm. The filtrate was filled into vials (2ml or 160 mg 5,10-CH₂-(6R)-THF*Na₂ per vial) while keeping the solution as cold as possible.

### Example 2: Preparation of a lyophilized composition comprising disodium 5,10-methylene-(6R)-tetrahydrofolate, citrate and sodium sulfate

The vials from Example 1 were frozen and subsequently freeze-dried. The vials were sealed under a slight vacuum with nitrogen in the headspace, and crimped. The resulting lyophilisate contains more than 30% w/w 5,10-CH₂-(6R)-THF*Na₂.

### Example 3: Stability testing

a) Stability of a solution comprising disodium 5,10-methylene-(6R)-tetrahydrofolic acid, citrate and sulfate at at 25°C
In order to determine the stability of a sterile filtered solution comprising disodium 5,10-methylene-(6R)-tetrahydrofolic acid, citrate and sulfate at 25°C, a solution as prepared in Example 1, step (d) was stored at 25°C. The purity of 5,10-CH₂-(6R)-THF was measured by HPLC at periodic intervals (% area). The results are shown in Figure 4.

During the investigation the color of the test solution darkened from yellow to dark yellow. No protection from light was used for samples kept at room temperature. At room temperature the purity remains stable at 96% over a 7-hour storage period.

The progression of analytical results during the stability testing are summarized in Figure 4.

The content of the main degradation product, 10-formyl-(6R)-tetrahydrofolic acid (10-FTHFA) when stored at 25°C was measured by HPLC at periodic intervals. The results are shown in Figure 5.

b) Stability of a solution comprising disodium 5,10-methylene-(6R)-tetrahydrofolic acid, citrate and sulfate at 5°C
In order to determine the stability of a sterile filtered solution comprising disodium 5,10-methylene-(6R)-tetrahydrofolic acid, citrate and sodium sulfate at 5°C a solution as prepared in Example 1, step d was stored at 5°C. The purity of 5,10-CH₂-(6R)-THF was measured by HPLC at periodic intervals (% area). The results are shown in Figure 4.

The content of the main degradation product, 10-formyl-(6R)-tetrahydrofolic acid (10-FTHFA) was measured by HPLC at periodic intervals. The results are shown in Figure 5.

During the investigation the color of the test solution darkened from yellow to dark yellow. The stability test solution was stored in a refrigerator (2°C to 8°C) during the investigation, and only taken out for analytical sampling. During the sampling no protection from light was used. At 2°C to 8°C the purity shows no significant change and remains stable at 97% over a 7-hour storage period.

The progression of analytical results during the stability testing are summarized in Figure 4 and Figure 5.

The stability test at 5°C was repeated on a comparative test solution to which was added sodium ascorbate (1mg/mL as antioxidant) and stored at 2°C to 8°C. The test solution with sodium ascorbate shows no significant difference compared to the test solution without added sodium ascorbate (Figure 4 and Figure 5). The solutions with and without ascorbate may thus be considered equal with regard to stability.

A solution of 75 mg/mL 5,10-methylene-(6R)-tetrahydrofolate is clear and remains clear regardless if it is stored at 2-8°C or at RT, i.e. no precipitation occurs. The solution is slightly viscous and has an osmolality of app. 710 mOsmol/kg.

In conclusion, a sterile filtered solution comprising 75 mg/mL disodium 5,10-methylene-(6R)-tetrahydrofolic acid, citrate and sulfate is stable up to 7 hours when stored at 2°C to 8°C, and stable up to 4 hours when stored at room temperature.

c) Stability of a lyophilized composition comprising disodium 5,10-methylene-(6R)-tetrahydrofolate, citrate and sodium sulfate at 5 °C, 25°C, and 40°C
In order to determine the stability of a lyophilized composition comprising disodium 5,10-methylene-(6R)-tetrahydrofolic acid, citrate and sulfate at at different temperatures over time, a liophilisate as prepared in Example 2 was stored at 5 °C, 25°C, and 40 °C. The purity of 5,10-CH₂-(6R)-THF was measured by HPLC at periodic intervals (% area). The results relative to the staring value are shown as relative purity in %w/w in Figure 6.

The content of the main degradation product, 10-formyl-(6R)-tetrahydrofolic acid (10-FTHFA) when stored at 5 °C, 25°C, and 40 °C was measured by HPLC at periodic intervals. The results are shown in Figure 7.

## Claims

1. A lyophilized composition which comprises the disodium salt of 5,10-methylene-(6R)-tetrahydrofolic acid, citrate and an alkali metal sulfate, which composition does not contain any further reducing agents or antioxidants, and wherein the concentration of said disodium salt of 5,10-methylene-(6R)-tetrahydrofolic acid is more than 30% w/w.

2. A lyophilized composition according to claim 1, which consists of the disodium salt of 5,10-methylene-(6R)-tetrahydrofolic acid, sodium sulfate, sodium citrate, water and optional osmolality correcting additives.

3. A lyophilized composition according to claim 1 or claim 2 wherein the molar ratio between the disodium salt of 5,10-methylene-(6R)-tetrahydrofolic acid and sulfate is from 40 to 200 mol%.

4. A lyophilized composition according to any one of claim 1 to 3, comprising 200-400 mol% citrate.

5. A lyophilized composition according to any one of claim 1 to 4, which is a stable lyophilizate with a concentration of the disodium salt of 5,10-methylene-(6R)-tetrahydrofolic acid of more than 35% w/w, or such as about 40 % w/w disodium salt of 5,10-methylene-(6R)-tetrahydrofolic acid.

6. A lyophilized composition according to any one of claim 1 to 5 containing the disodium salt of 5,10-methylene-(6R)-tetrahydrofolic acid of a purity greater than 98%.

7. A reconstituted product obtained by dissolving the lyophilized composition of any one of claims 1 to 6 in water or a liquid pharmaceutically acceptable vehicle.

8. A reconstituted product according to claim 7, wherein the water is sterile water for injection.

9. A reconstituted product according to any one of claims 7 or 8, further comprising a pharmaceutically acceptable carrier.

10. A reconstituted product according to any one of claims 7 to 9, further comprising an additional pharmaceutically acceptable active ingredient.

11. A reconstituted product according to any one of claims 7 to 10, further comprising a buffer and/or one or more osmolality correcting excipients.

12. A reconstituted product according to any one of claims 7 to 11 for use in the treatment of cancer or in cancer therapy.

13. A process for the preparation of a lyophilized composition according to any one of claims 1 to 6 comprising the disodium salt of 5,10-methylene-(6R)-tetrahydrofolic acid, citrate and an alkali metal sulfate, which process comprises the following steps:
i. dissolving (6S)-tetrahydrofolic acid in water at pH 11,
ii. adjusting the pH of the clear solution to 8.6 ± 0.5,
iii. adding 110 - 120 mol% formaldehyde,
iv. stirring the reaction mixture until reaction has completed,
v. adding from 40 - 200 mol-% of an alkali metal sulfate and 100 - 400 mol-% citrate,
vi. filtering the reaction mixture to obtain a clear solution, and
vii. freeze-drying the obtained clear solution,
which process does not comprise adding any further reducing agents or antioxidants, and wherein the concentration of said disodium salt of 5,10-methylene-(6R)-tetrahydrofolic acid in the lyophilized composition is more than 30% w/w.

14. A process according to claim 13 wherein the alkali metal sulfate is added in the form of sodium sulfate.

15. A process according to claims 13 or 14 wherein citrate is added as citric acid or sodium citrate.

## Patentansprüche

1. Lyophilisierte Zusammensetzung, die das Dinatriumsalz von 5,10-Methylen-(6R)-tetrahydrofolsäure, Citrat und ein Alkalimetallsulfat umfasst, wobei die Zusammensetzung keine weiteren Reduktionsmittel oder Antioxidantien enthält und wobei die Konzentration des Dinatriumsalzes von 5,10-Methylen-(6R)-tetrahydrofolsäure mehr als 30 Gew.-% beträgt.

2. Lyophilisierte Zusammensetzung nach Anspruch 1, bestehend aus dem Dinatriumsalz von 5,10-Methylen-(6R)-tetrahydrofolsäure, Natriumsulfat, Natriumcitrat, Wasser und fakultativen osmolalitätskorrigierenden Zusätzen.

3. Lyophilisierte Zusammensetzung nach Anspruch 1 oder Anspruch 2, wobei das Molverhältnis zwischen dem Dinatriumsalz von 5,10-Methylen-(6R)-tetrahydrofolsäure und Sulfat 40 bis 200 Mol-% beträgt.

4. Lyophilisierte Zusammensetzung nach einem der Ansprüche 1 bis 3, umfassend 200 - 400 Mol-% Citrat.

5. Lyophilisierte Zusammensetzung nach einem der Ansprüche 1 bis 4, bei der es sich um ein stabiles Lyophilisat mit einer Konzentration des Dinatriumsalzes von 5,10-Methylen-(6R)-tetrahydrofolsäure von mehr als 35 Gew.-% oder etwa 40 Gew.-% Dinatriumsalz von 5,10-Methylen-(6R)-tetrahydrofolsäure handelt.

6. Lyophilisierte Zusammensetzung nach einem der Ansprüche 1 bis 5, die das Dinatriumsalz von 5,10-Methylen-(6R)-tetrahydrofolsäure mit einer Reinheit von mehr als 98 % enthält.

7. Rekonstituiertes Produkt, erhalten durch Auflösen der lyophilisierten Zusammensetzung nach einem der Ansprüche 1 bis 6 in Wasser oder einem flüssigen pharmazeutisch akzeptablen Vehikel.

8. Rekonstituiertes Produkt nach Anspruch 7, wobei das Wasser steriles Wasser zur Injektion ist.

9. Rekonstituiertes Produkt nach einem der Ansprüche 7 oder 8, das ferner einen pharmazeutisch akzeptablen Träger umfasst.

10. Rekonstituiertes Produkt nach einem der Ansprüche 7 bis 9, das ferner einen zusätzlichen pharmazeutisch akzeptablen Wirkstoff umfasst.

11. Rekonstituiertes Produkt nach einem der Ansprüche 7 bis 10, das ferner einen Puffer und/oder einen oder mehrere osmolalitätskorrigierende Hilfsstoffe umfasst.

12. Rekonstituiertes Produkt nach einem der Ansprüche 7 bis 11 zur Verwendung bei der Behandlung von Krebs oder in der Krebstherapie.

13. Verfahren zur Herstellung einer lyophilisierten Zusammensetzung nach einem der Ansprüche 1 bis 6, umfassend das Dinatriumsalz von 5,10-Methylen-(6R)-tetrahydrofolsäure, Citrat und ein Alkalimetallsulfat, wobei das Verfahren die folgenden Schritte umfasst:
i. Lösen von (6S)-Tetrahydrofolsäure in Wasser bei pH 11,
ii. Einstellen des pH-Wertes der klaren Lösung auf 8,6 ± 0,5,
iii. Zugabe von 110 - 120 Mol-% Formaldehyd,
iv. Rühren der Reaktionsmischung, bis die Reaktion abgeschlossen ist,
v. Zugabe von 40 - 200 Mol-% eines Alkalimetallsulfats und 100 - 400 Mol-% Citrat,
vi. Filtrieren der Reaktionsmischung, um eine klare Lösung zu erhalten, und
vii. Gefriertrocknen der erhaltenen klaren Lösung,
wobei das Verfahren nicht die Zugabe weiterer Reduktionsmittel oder Antioxidantien umfasst und wobei die Konzentration des Dinatriumsalzes von 5,10-Methylen-(6R)-tetrahydrofolsäure in der lyophilisierten Zusammensetzung mehr als 30 Gew.-% beträgt.

14. Verfahren nach Anspruch 13, wobei das Alkalimetallsulfat in Form von Natriumsulfat zugegeben wird.

15. Verfahren nach Anspruch 13 oder 14, wobei Citrat als Zitronensäure oder Natriumcitrat zugegeben wird.

## Revendications

1. Composition lyophilisée comprenant le sel disodique de l'acide 5,10-méthylène-(6R)-tétrahydrofolique, le citrate et un sulfate de métal alcalin, cette composition ne contenant pas d'autres agents réducteurs ou antioxydants, et dans laquelle la concentration dudit sel disodique de l'acide 5,10-méthylène-(6R)-tétrahydrofolique est supérieure à 30 % p/p.

2. Composition lyophilisée selon la revendication 1, qui est constituée du sel disodique de l'acide 5,10-méthylène-(6R)-tétrahydrofolique, de sulfate de sodium, de citrate de sodium, d'eau et d'additifs facultatifs de rectification de l'osmolalité.

3. Composition lyophilisée selon la revendication 1 ou la revendication 2 dans laquelle le rapport molaire entre le sel disodique de l'acide 5,10-méthylène-(6R)-tétrahydrofolique et le sulfate est de 40 à 200 mol%.

4. Composition lyophilisée selon l'une des revendications 1 à 3, comprenant 200-400 mol% de citrate.

5. Composition lyophilisée selon l'une des revendications 1 à 4, qui est un lyophilisat stable avec une concentration du sel disodique de l'acide 5,10-méthylène-(6R)-tétrahydrofolique de plus de 35 % p/p, ou tel qu'environ 40 % p/p de sel disodique de l'acide 5,10-méthylène-(6R)-tétrahydrofolique.

6. Composition lyophilisée selon l'une des revendications 1 à 5 contenant le sel disodique de l'acide 5,10-méthylène-(6R)-tétrahydrofolique d'une pureté supérieure à 98%.

7. Produit reconstitué obtenu par dissolution de la composition lyophilisée de l'une quelconque des revendications 1 à 6 dans de l'eau ou dans un véhicule liquide pharmaceutiquement acceptable.

8. Produit reconstitué selon la revendication 7, dans lequel l'eau est de l'eau stérile pour injection.

9. Produit reconstitué selon l'une quelconque des revendications 7 ou 8, comprenant en outre un support pharmaceutiquement acceptable.

10. Produit reconstitué selon l'une des revendications 7 à 9, comprenant en outre un principe actif supplémentaire pharmaceutiquement acceptable.

11. Produit reconstitué selon l'une des revendications 7 à 10, comprenant en outre un tampon et/ou un ou plusieurs excipients corrigeant l'osmolalité.

12. Produit reconstitué selon l'une quelconque des revendications 7 à 11 pour l'utilisation dans le traitement du cancer ou dans la thérapie du cancer.

13. Procédé de préparation d'une composition lyophilisée selon l'une quelconque des revendications 1 à 6, comprenant le sel disodique de l'acide 5,10-méthylène-(6R)-tétrahydrofolique, le citrate et un sulfate de métal alcalin, lequel procédé comprend les étapes suivantes :
i. dissolution de l'acide (6S)-tétrahydrofolique dans l'eau à un pH de 11,
ii. ajustement du pH de la solution claire à 8,6 ± 0,5,
iii. ajout de 110 - 120 mol% de formaldéhyde,
iv. agitation du mélange réactionnel jusqu'à ce que la réaction soit terminée,
v. ajout de 40 à 200 % en moles d'un sulfate de métal alcalin et de 100 à 400 % en moles de citrate,
vi. filtration du mélange réactionnel pour obtenir une solution claire, et
vii. lyophilisation de la solution claire obtenue,
ce procédé ne comprend pas l'ajout d'autres agents réducteurs ou antioxydants, et dans lequel la concentration dudit sel disodique de l'acide 5,10-méthylène-(6R)-tétrahydrofolique dans la composition lyophilisée est supérieure à 30 % p/p.

14. Procédé selon la revendication 13 dans lequel le sulfate de métal alcalin est ajouté sous forme de sulfate de sodium.

15. Procédé selon les revendications 13 ou 14, dans lequel le citrate est ajouté sous forme d'acide citrique ou de citrate de sodium.
